# EUROPEAN PATENT SPECIFICATION

(11) **EP 3 040 055 B1**
(45) Date of publication and mention of the grant of the patent: **23.05.2018**
(21) Application number: 15198073.7
(22) Date of filing: 04.12.2015
(51) Int. Cl.: A61F 2/95, A61F 2/966

(54) **CRIMPING METHOD AND CRIMPING STRUCTURE FOR ENDOVASCULAR STENT**
CRIMPVERFAHREN UND CRIMPANORDNUNG FÜR ENDOVASKULÄREN STENT
PROCÉDÉ ET STRUCTURE DE SERTISSAGE DE STENT ENDOVASCULAIRE

(30) Priority: 30.12.2014 CN 201410854547
(43) Date of publication of application: 06.07.2016
(73) Proprietor: Shanghai Bio-Heart Biological Technology Co., Ltd., Pudong District, Shanghai 201201 (CN); Fu Wai Hospital, Cams &Pumc, Xicheng District, Beijing 100037 (CN)
(72) Inventor: ZHAO, Yinghong, 201201 Pudong District Shanghai (CN); WU, Yongjian, 201201 Pudong District Shanghai (CN); XU, Bo, 201201 Pudong District Shanghai (CN)
(74) Representative: HGF Limited

(56) References cited:
- EP-A2- 1 226 798
- WO-A1-01/21110
- WO-A2-2012/027172
- US-A1- 2012 010 693

## Description

### TECHNICAL FIELD

The present disclosure relates to the field of medical devices and its manufacture, in particular to a crimping method and a crimping structure for an endovascular stent.

### BACKGROUND

In the related art, usually a to-be-crimped stent is covered with a liner and then curled onto a balloon, so that the to-be-crimped stent is directly crimped through the support from the balloon However, it is unable to ensure an entire outer surface of the to-be-crimped stent is completely covered with the liner, and during the crimping, the uncovered stent may be deformed due to uneven forces applied thereto. In addition, the balloon is made of a soft material, and an insufficient force is applied by the balloon so as to support the to-be-crimped stent. As a result, the to-be-crimped stent may be folded, and even broken, during the crimping. A crimping method for an endovascular stent of the type set forth in the preamble of claim 1, and a crimping structure for an endovascular stent of the type set forth in the preamble of the accompanying claim 12 is known from EP 1226798

WO 2012/027172 A2 discloses a medical device including a polymer scaffold crimped to a catheter having an expansion balloon. The scaffold is crimped to the catheter by a multi-step process for increasing scaffold-catheter yield following a crimping sequence. Damage reduction during a crimping sequence includes modifying blades of a crimper, adopting a multi-step crimping sequence, and inflating a supporting balloon to support the scaffold during crimping.

### SUMMARY

An object of the present disclosure is to provide a crimping method and a crimping structure for an endovascular stent, as disclosed in the appended claims, so as to prevent a to-be-crimped stent from being damaged, and even broken, due to uneven forces applied thereto. Insofar as the term "invention" and or "embodiment" are used in the following, and/or features are presented as being optional, this should be interpreted in such a way that the only protection sought is that of the invention as claimed.

In one aspect, the present disclosure provides in one embodiment a crimping method for an endovascular stent, including the steps of preparing a crimping structure including a to-be-crimped stent, a supporting mandrel arranged inside the to-be-crimped stent, and a stopping pipe sleeved onto the to-be-crimped stent; and crimping the crimping structure in accordance with a first crimping configuration, until the to-be-crimped stent is attached to the supporting mandrel and a force value from a crimper reaches a first value. The supporting mandrel is of a solid, cylindrical structure.

The step of preparing the crimping structure includes: selecting the supporting mandrel and the stopping pipe fit to the to-be-crimped stent with a diameter of the supporting mandrel being a percentage of a current diameter of the to-be-crimped stent; and sleeving the to-be-crimped stent onto the supporting mandrel, and sleeving the stopping pipe onto the to-be-crimped stent with the stopping pipe being attached to the to-be-crimped stent.

Subsequent to crimping the crimping structure, the crimping method further includes: removing the to-be-crimped stent which has been crimped from the supporting mandrel and the stopping pipe, to obtain the to-be-crimped stent which has been crimped.

Subsequent to removing the to-be-crimped stent which has been crimped between the supporting mandrel and the stopping pipe, the crimping method further includes: sleeving the to-be-crimped stent which has been crimped onto a balloon catheter and sleeving another stopping pipe onto the to-be-crimped stent which has been crimped, so as to prepare a delivery structure; and inflating the balloon catheter in accordance with a second crimping configuration, and crimping the delivery structure, until the to-be-crimped stent which has been crimped is secured onto the balloon catheter and the force value from the crimper reaches a second value.

Before sleeving the to-be-crimped stent which has been crimped onto the balloon catheter to prepare the delivery structure, the crimping method further includes: repeating the steps of preparing a crimping structure and crimping the crimping structure, until the diameter of the to-be-crimped stent is reduced to a predetermined value.

Subsequent to crimping the delivery structure, the crimping method further includes removing the stopping pipe from the to-be-crimped stent which has been crimped.

The diameter of the supporting mandrel is 60% to 90% of the diameter of the to-be-crimped diameter.

The step of crimping the crimping structure in accordance with the first crimping configuration includes: setting the first crimping configuration for the crimper including a first preheating temperature, a first preheating duration, a first diameter of a crimping hole, a first diameter reduction rate of the crimping hole, the first value and a first form maintenance duration; and crimping the crimping structure in accordance with the first crimping configuration.

The first preheating temperature is 45°C to 55°C; the first preheating duration is 30s to 120s; the first diameter is identical to the current diameter of the to-be-crimped stent; the first diameter reduction rate is 0.01mm/s to 0.2mm/s; the first value is 10N to 60N; the first form maintenance duration is 30s to 120s.

The steps of inflating the balloon catheter in accordance with the second crimping configuration and crimping the delivery structure includes: setting the second crimping configuration for the crimper including a second preheating temperature, a second preheating duration, a second diameter of the crimping hole, a second diameter reduction rate of the crimping hole, a filling pressure of the balloon catheter, the second value, and a second form maintenance duration; and inflating the balloon catheter in accordance with the second crimping configuration and crimping the delivery structure.

The second preheating temperature is 45°C to 55°C; the second preheating duration is 30s to 120s; the second diameter is identical to the current diameter of the to-be-crimped stent; the second diameter reduction rate is 0.01mm/s to 0.2mm/s; the filling pressure of the balloon catheter is 207kPa (30psi) to 1380kPa (200psi); the second value is 100N to 150N; the second form maintenance duration is 60s to 240s.

In another aspect, the present disclosure provides in one embodiment a crimping structure for an endovascular stent, including a tubular to-be-crimped stent, a supporting mandrel onto which the to-be-crimped stent is sleeved, and a stopping pipe capable of being deformed by an external force, and sleeved onto and being attached to the to-be-crimped stent. A diameter of the supporting mandrel is a percentage of a diameter of the to-be-crimped stent. The supporting mandrel is of a solid, cylindrical structure.

Preferably, the percentage is 60% to 90%.

Preferably, the supporting mandrel is made of polytetrafluoroethylene (PTFE), polyamide fiber (Nylon) or polyether block amide (PEBAX).

Preferably, a friction coefficient of an outer surface of the supporting mandrel is less than a threshold.

Preferably, the stopping pipe is made of PTFE, Nylon or PEBAX.

Preferably, axial lengths of each of the supporting mandrel and the stopping pipe are greater than an axial length of the to-be-crimped stent.

According to the crimping method and crimping structure for the endovascular stent in the embodiments of the present disclosure, the supporting mandrel is arranged inside the to-be-crimped stent, and the stopping pipe is sleeved onto the to-be-crimped stent. During the crimping, the to-be-crimped stent is internally supported by the supporting mandrel, so as to prevent the to-be-crimped stent from being damaged and accurately control the diameter of the to-be-crimped stent after being crimped. In addition, the to-be-crimped stent is stopped and protected by the stopping pipe, so as to ensure an even force applied to the to-be-crimped stent during the crimping, thereby to further prevent the to-be-crimped stent from being damaged from the outside. As a result, it is able to protect the to-be-crimped stent from both the inside and the outside, thereby to remarkably improve the crimping efficiency and the success rate.

### BRIEF DESCRIPTION OF THE DRAWINGS

Fig.1 is a flow chart of a crimping method for an endovascular stent according to one embodiment of the present disclosure;
Fig.2 is another flow chart of the crimping method for an endovascular stent according to one embodiment of the present disclosure;
Fig.3 is a flow chart of a method for preparing a crimping structure in Fig.1 or 2;
Fig.4 is a schematic view showing the crimping structure according to one embodiment of the present disclosure;
Fig.5 is a sectional view of the crimping structure in Fig.4;
Fig.6 is a schematic view showing a to-be-crimped stent before being crimped; and
Fig.7 is a schematic view showing the to-be-crimped stent after being crimped.

### Reference sign list

- 1: to-be-crimped stent
- 2: supporting mandrel
- 3: stopping pipe

### DETAILED DESCRIPTION

The present disclosure will be described hereinafter in conjunction with the drawings and embodiments. Obviously, the following embodiments are for illustrative purposes only, but shall not be used to limit the scope of the present disclosure.

### Embodiments

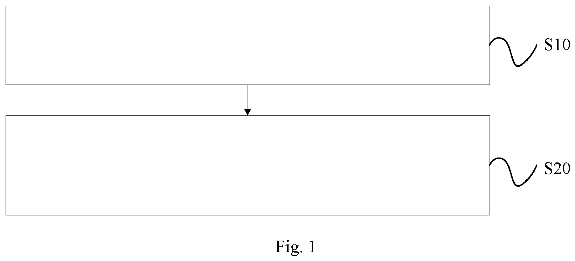

As shown in Fig.1, the present disclosure provides in one embodiment a crimping method for an endovascular stent, which includes the following steps.

Step S10: preparing a crimping structure, which includes a to-be-crimped stent, a supporting mandrel arranged inside the to-be-crimped stent, and a stopping pipe sleeved onto the to-be-crimped stent.

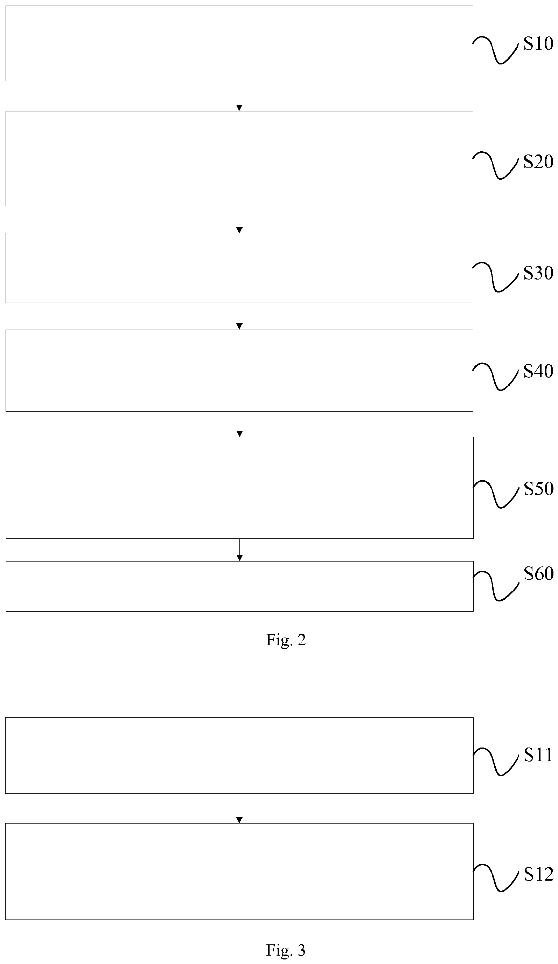

This step of preparing the crimping structure may be carried out as shown in Fig.3.

Step S11: selecting the supporting mandrel and the stopping pipe fit to the to-be-crimped stent with a diameter of the supporting mandrel being a predetermined percentage of a current diameter of the to-be-crimped stent.

The supporting mandrel functions as to support the to-be-crimped stent from the inside and provide a reference for a diameter reduction amount. The diameter reduction amount is limited as being not more than 40% so as to ensure a stress relaxation characteristic of a polymer material, so the diameter of the selected supporting mandrel is not less than 60% of the current diameter of the to-be-crimped stent. Preferably, the diameter of the supporting mandrel is 60% to 90% of the current diameter of the to-be-crimped stent. Especially, the diameter of the supporting mandrel is 80% of the current diameter of the to-be-crimped stent, i.e., the diameter reduction amount of the to-be-crimped stent is 20%.

Step S12: sleeving the to-be-crimped stent onto the supporting mandrel, and sleeving the stopping pipe onto the to-be-crimped stent with the stopping pipe being attached to the to-be-crimped stent.

In order to ensure an even force applied onto an inner surface and an outer surface of the to-be-crimped stent during the crimping, the supporting mandrel is arranged inside the to-be-crimped stent, and the stopping pipe is sleeved onto the to-be-crimped stent, so as to form the crimping structure. The supporting mandrel is of relatively large strength, so it is able to support the to-be-crimped stent with a relatively large force and prevent the to-be-crimped stent from being folded easily during the crimping, thereby to prevent the to-be-crimped stent from being damaged or deformed. In addition, in order to protect the outer surface of the to-be-crimped stent from being damaged, it is ensured that the stopping pipe sleeved onto the to-be-crimped stent is attached to the to-be-crimped stent. In this way, the to-be-crimped stent is enclosed by the stopping pipe, and an even force is applied to the to-be-crimped stent during the crimping, so it is able to prevent the occurrence of wrinkles.

Step S20: crimping the crimping structure in accordance with a first predetermined crimping configuration, until the to-be-crimped stent is attached to the supporting mandrel and a force value from a crimper reaches a first predetermined value.

After the crimping structure has been prepared, it is needed to set for the crimper the first crimping configuration which includes a first preheating temperature, a first preheating duration, a first diameter of a crimping hole, a first diameter reduction rate of the crimping hole, the first predetermined value and a first form maintenance duration. Usually, a glass transition temperature (TG) of a polymer is about 45°C to 55°C, so the first preheating temperature may be 45°C to 55°C, and preferably, 50°C. The first preheating duration may be 30s to 120s, and preferably, 90s. The prepared crimping structure is placed into the crimping hole of the crimper, and then the first diameter of the crimping hole may be set. Usually, the first diameter of the crimping hole is identical to a current diameter of the crimping structure, i.e., the crimping structure is attached to the crimping hole. During the crimping, the crimping structure may not be crimped by the crimper too fast or too slowly. In the case of the former, the to-be-crimped stent may be damaged or even broken due to an uneven force applied thereto, and in the case of the latter, the crimping may be failed due to a decrease in the temperature. Usually, the first diameter reduction rate may be 0.01mm/s to 0.2mm/s, and preferably, 0.05mm/s. After the inner surface of the to-be-crimped stent is attached to the supporting mandrel during the crimping, i.e., after the reduction amount of the to-be-crimped stent reaches a predetermined value, the crimping needs to be stopped when a force value from the crimper (i.e., the force applied by the crimper to the to-be-crimped stent) reaches the first predetermined value. Empirically, the first predetermined value may be 10N to 60N, and preferably, 40N, so as to overcome the internal stress released by the polymer stent after it is deformed. This force value may be maintained for the first form maintenance duration, so as to shape the to-be-crimped stent. Usually, the first form maintenance duration may be 30s to 120s, and preferably, 60s. After setting these parameters, the crimping structure may be crimped in accordance with the first crimping configuration. In this way, it is able to ensure an even force applied to the to-be-crimped stent during the crimping and prevent the to-be-crimped stent from being damaged or deformed, thereby to improve the crimping efficiency and the success rate.

The diameter reduction amount of the to-be-crimped stent is 20% for each crimping procedure, but a larger diameter reduction amount may be applied during the actual application, so it is needed to crimp the to-be-crimped stent several times, so as to realize a larger diameter reduction amount. In this case, it is needed to repeat Steps S10 and S20. It should be appreciated that, the selection of the supporting mandrel and the stopping pipe, as well as the setting of the first crimping configuration for each crimping procedure may follow the above principles, and will not be repeated herein.

As shown in Fig.2, after the crimping of the to-be-crimped stent has been finished, the crimping method further includes the following steps.

Step S30: removing the to-be-crimped stent which has been crimped between the supporting mandrel and the stopping pipe, so as to obtain the to-be-crimped stent which has been crimped.

After finishing each crimping procedure and after being maintained for the first form maintenance duration, it is needed to remove the stopping pipe from the to-be-crimped stent and pull the supporting mandrel out from the to-be-crimped stent, so as to obtain the to-be-crimped stent which has been crimped.

Step S40: sleeving the to-be-crimped stent which has been crimped onto a balloon catheter and sleeving another stopping pipe onto the to-be-crimped stent, so as to form a delivery structure.

In medical applications, the endovascular stent is delivered to a lesion area with the balloon catheter, so it is needed to secure the endovascular stent is crimped onto the balloon catheter, i.e., to prepare the delivery structure. In order to ensure the integrity of the outer and inner surfaces of the to-be-crimped stent, the delivery structure may be constructed with reference to structures of the above-mentioned crimping structure.

Step S50: inflating the balloon catheter in accordance with a second predetermined crimping configuration, and crimping the delivery structure, until the to-be-crimped stent is secured onto the balloon catheter and a force value from the crimper reaches a second predetermined value.

In this step, the balloon catheter is arranged inside the crimped stent and then inflated, so as to enable the to-be-crimped stent to be attached to the balloon catheter. Although the to-be-crimped stent is crimped by the crimper, the diameter of the to-be-crimped stent may not be reduced remarkably. Before this step, it is also needed to set for the crimper the second crimping configure which includes such parameters as a second preheating temperature, a second preheating duration, a second diameter of the crimping hole, a second diameter reduction rate of the crimping hole, a filling pressure of the balloon catheter, the second predetermined value and a second form maintenance duration. The setting procedure of the second crimping configuration is similar to that of the first crimping configuration, with difference in values of some parameters. For example, in the second crimping configuration, the second diameter reduction rate is 0.01mm/s to 0.2mm/s, and preferably, 0.03mm/s; the filling pressure of the balloon catheter is 207 kPa (30 psi) to 1380 kPa (200psi), and preferably, 550 kPa (80psi); the second value is 100N to 150N, and preferably, 120N; and the second form maintenance duration is 60s to 240s, and preferably, 180s.

Step S60: removing the stopping pipe from the to-be-crimped stent.
In a word, according to the crimping method for the endovascular stentin embodiments of the present disclosure, through the special crimping structure, it is able to prevent the to-be-crimped stent from being damaged from both the inside and the outside during the crimping, thereby to improve the crimping efficiency and the success rate.

In a word, according to the crimping method for the endovascular stent in embodiments of the present disclosure, through the special crimping structure, it is able to prevent the to-be scrimped stent from being damaged from both the inside and the outside during the crimping, thereby to improve the crimping efficiency and the success rate.

For ease of understanding, the following two examples will be provided for illustrating the crimping method.

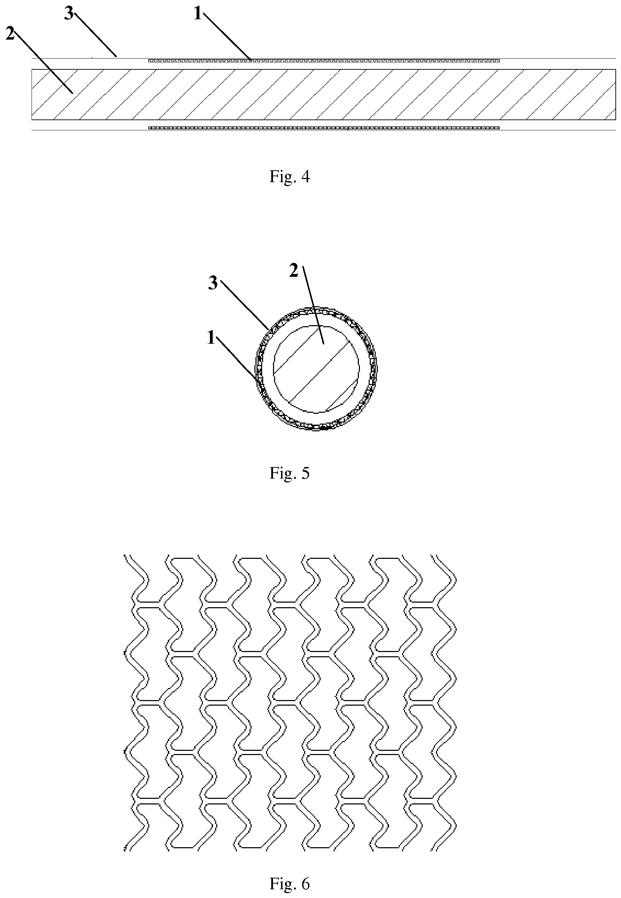

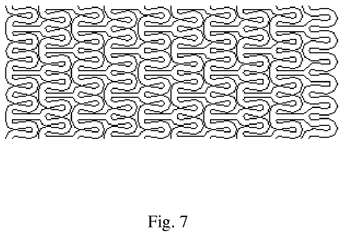

Example 1: in this example, the to-be-crimped stent is crimped once. As shown in Fig.6, before the crimping, the to-be-crimped stent has an outer diameter of 3mm, an inner diameter of 2.7mm, a thickness of 0.15mm, and a length of 15mm. As shown in Fig.7, after the crimping, the to-be-crimped stent expectedly has an outer diameter of 2.5mm and an inner diameter of 2.2mm.

In order to ensure the crimping quality, once upon each crimping procedure has been completed, the diameter of the to-be-crimped stent may be reduced by 20%. As shown in Figs.4 and 5, the selected supporting mandrel 2 has a diameter of 2.2mm, the to-be-crimped stent 1 having an outer diameter of 3mm is sleeved onto the supporting mandrel 2, and the stopping pipe 3 having an inner diameter of 3.1 mm or a value closer to the outer diameter of the to-be-crimped stent is sleeved onto the to-be-crimped stent 1, so as to prepare a crimping structure.

The prepared crimping structure is placed into the crimping hole of the crimper, and then the diameter of the crimping hole is adjusted to 3mm, so as to hold the crimping structure tightly and enable the stopping pipe to be attached to the to-be-crimped stent.

After the first crimping configuration has been set, the crimping procedure is started. To be specific, the crimping structure is preheated for 90s at the first preheating temperature of 50°C, and then crimped at the first diameter reduction rate of 0.05mm/s. During the crimping, the diameter of the to-be-crimped stent decreases gradually, until the to-be-crimped stent is attached to the supporting mandrel. At this time, the crimper does not cease the application of the force to the crimping structure, until the force value from the crimper reaches the first predetermined value of 40N. However, the internal stress may be released by the to-be-crimped stent after it is deformed, so it is needed to maintain the crimping for the first form maintenance duration of 60s. After 60s, the inner stress is substantially released, and at this time, the crimping structure is removed from the crimping hole.

Then, by using tweezers, the stopping pipe is removed from the to-be-crimped stent and the supporting mandrel is pulled out from the to-be-crimped stent. In order to prevent the to-be-crimped stent from being damaged during the removal, it is needed to ensure that the lengths of the supporting mandrel and the stopping pipe are larger than, usually 5mm larger than, that of the to-be-crimped stent.

When the diameter of the to-be-crimped stent which has been crimped reaches an expected value after this crimping procedure, the to-be-crimped stent which has been crimped is sleeved onto the balloon catheter, and another stopping pipe is sleeved onto the to-be-crimped stent which has been crimped, so as to form the delivery structure.

The prepared delivery structure is placed into the crimping hole of the crimper, and then the diameter of the crimping hole is adjusted to 2.5mm, so as to hold the delivery structure tightly and enable the stopping pipe to be attached to the to-be-crimped stent.

After the second crimping configuration has been set, the balloon catheter is inflated and the delivery structure is crimped. To be specific, the delivery structure is preheated for 90s at the second preheating temperature of 50°C, and then crimped at the second diameter reduction rate of 0.03mm/s. Meanwhile, the balloon catheter is inflated so as to obtain the filling pressure of 80psi. During the crimping, the diameter of the to-be-crimped stent is almost not reduced. The inflation and the crimping are not stopped until the force value from the crimper reaches the second predetermined value of 120N. However, the internal stress may be released by the to-be-crimped stent after it is deformed, so it is needed to maintain the crimping for the second form maintenance duration of 180s. After 180s, the internal stress is substantially released, and at this time, the delivery structure is removed from the crimping hole.

Then, by using tweezers, the stopping pipe is removed from the to-be-crimped stent, and the entire crimping procedure is completed.

Example 2: in this example, the to-be-crimped stent is crimped several times. Before the crimping, the to-be-crimped stent has an outer diameter of 3mm, an inner diameter of 2.7mm, a thickness of 0.15mm, and a length of 15mm. After the crimping, the to-be-crimped stent expectedly has an outer diameter of 1.9mm and an inner diameter of 1.6mm.

Primary crimping: in order to ensure the crimping quality, after each crimping procedure, the diameter of the to-be-crimped stent is reduced by 20%. In this case, the selected supporting mandrel has a diameter of 2.2mm, the to-be-crimped stent having an outer diameter of 3mm is sleeved onto the supporting mandrel, and the stopping pipe having an inner diameter of 3.1mm or a value closer to the outer diameter of the to-be-crimped stent is sleeved onto the to-be-crimped stent, so as to prepare a crimping structure.

The prepared crimping structure is placed into the crimping hole of the crimper, and then the diameter of the crimping hole is adjusted to 3mm, so as to hole the crimping structure tightly and enable the stopping pipe to be attached to the to-be-crimped stent.

After the first crimping configuration has been set, the crimping procedure is started. To be specific, the crimping structure is preheated for 90s at the first preheating temperature of 50°C, and then crimped at the first diameter reduction rate of 0.05mm/s. During the crimping, the diameter of the to-be-crimped stent decreases gradually, until the to-be-crimped stent is attached to the supporting mandrel. At this time, the crimper does not cease the application of the force to the crimping structure, until the force value from the crimper reaches the first predetermined value of 40N. However, the internal stress may be released by the to-be-crimped stent after it is deformed, so it is needed to maintain the crimping for the first form maintenance duration of 60s. After 60s, the inner stress is substantially released, and at this time, the crimping structure is removed from the crimping hole.

Then, by using tweezers, the stopping pipe is removed from the to-be-crimped stent and the supporting mandrel is pulled out from the to-be-crimped stent. In order to prevent the to-be-crimped stent from being damaged during the removal, it is needed to ensure that the lengths of the supporting mandrel and the stopping pipe are larger than, usually 5mm larger than, that of the to-be-crimped stent.

When the diameter of the to-be-crimped stent which has been crimped is larger than an expected value after this crimping procedure, a secondary crimping is performed.

The secondary crimping: a supporting mandrel having a diameter of 1.6mm is selected, the to-be-crimped stent which has been crimped to have an inner diameter of 2.2mm is sleeved onto the supporting mandrel, and another stopping pipe having an inner diameter of 2.6mm or a value closer to an outer diameter of the to-be-crimped stent which has been crimped is sleeved onto the to-be-crimped stent, so as to prepare another crimping structure.

The prepared crimping structure is placed into the crimping hole of the crimper, and then the diameter of the crimping hole is adjusted to 2.6mm, so as to hold the crimping structure tightly and enable the stopping pipe to be attached to the to-be-crimped stent.

The crimping is started in accordance with the first crimping configuration, until the force value from the crimper reaches the first predetermined value of 40N. Then, the crimping is maintained for the first form maintenance duration of 60s. After 60s, the internal stress of the to-be-crimped stent is substantially released, and at this time, the crimping structure is removed from the crimping hole.

Then, by using tweezers, the stopping pipe is removed from the to-be-crimped stent, and the supporting mandrel is pulled out from the to-be-crimped stent. At this time, the to-be-crimped stent has an inner diameter of 1.6mm and an outer diameter of 1.9mm, i.e., it is reduced by the expected value. Next, the delivery structure may be prepared so as to complete the entire crimping.

According to the crimping method in the embodiments of the present disclosure, the supporting mandrel is arranged inside the to-be-crimped stent, and the stopping pipe is sleeved onto the to-be-crimped stent. During the crimping, the to-be-crimped stent is internally supported by the supporting mandrel, so as to prevent the to-be-crimped stent from being damaged and accurately control the diameter of the to-be-crimped stent after being crimped. In addition, the to-be-crimped stent is stopped and protected by the stopping pipe, so as to ensure an even force applied to the to-be-crimped stent during the crimping, thereby to further prevent the to-be-crimped stent from being damaged from the outside. As a result, it is able to protect the to-be-crimped stent from both the inside and the outside, thereby to remarkably improve the crimping efficiency and the success rate.

As shown in Figs.4 and 5, the present disclosure further provides in one embodiment a crimping structure for an endovascular stent, which includes a tubular to-be-crimped stent 1, a supporting mandrel 2, and a stopping pipe 3 capable of being deformed under the effect of an external force. The to-be-crimped stent 1 is sleeved onto the supporting mandrel 2, and the stopping pipe 3 is sleeved onto the to-be-crimped stent 1. In order to protect the to-be-crimped stent 1 by the stopping pipe 3 in a better manner and apply an even force onto the to-be-crimped stent during the crimping, the stopping pipe 3 is needed to be attached to the to-be-crimped stent 1.

In order to apply an even force onto the outer and inner surfaces of the to-be-crimped stent 1 during the crimping, the supporting mandrel 2 is arranged inside the to-be-crimped stent 1, and the stopping pipe 3 is sleeved onto the to-be-crimped stent 1, so as to form the crimping structure. The supporting mandrel 2 is of relatively large strength, so it is able to support the to-be-crimped stent 1 with a relatively large force and prevent the to-be-crimped stent 1 from being folded easily during the crimping, thereby to prevent the to-be-crimped stent 1 from being damaged or deformed. In addition, the to-be-crimped stent 1 is enclosed by the stopping pipe 3, and an even force is applied to the to-be-crimped stent 1 during the crimping, so it is able to prevent the occurrence of wrinkles. In a word, through the mentioned crimping structure, it is able to apply an even force to the to-be-crimped stent during the crimping and prevent the to-be-crimped stent from being damaged or deformed, thereby to improve the crimping efficiency and the success rate.

Preferably, as shown in Fig.4, in order to control a reduction amount of the to-be-crimped stent 1, a diameter of the supporting mandrel 2 is a predetermined percentage of a diameter of the to-be-crimped stent 1. When a radial force is applied to the stopping pipe 3 from the outer surface, the to-be-crimped stent 1 is squeezed inwardly by the stopping pipe 3, until the to-be-crimped stent 1 is attached to the supporting mandrel 2, i.e., a current diameter of the to-be-crimped stent 1 is identical to that of the supporting mandrel 2. It is difficult to ensure the crimping quality when the deformation is large, so when the deformation of the to-be-crimped stent is relatively large, preferably, the to-be-crimped stent may be crimped several times and the deformation generated during each crimping procedure may be controlled within an appropriate range. Experiments show that, when a diameter reduction amount of the to-be-crimped stent is 10% to 40%, it is able to ensure the crimping quality. Hence, the diameter of the supporting mandrel 2 may be 60% to 90% of the diameter of the to-be-crimped stent 1.

As shown in Fig.4, in order to remove the to-be-crimped stent 1 which has been crimped from the supporting mandrel 2 conveniently, there are certain requirements on a material and a friction coefficient of the supporting mandrel 2. Preferably, the supporting mandrel 2 may be made of a polymer material identical or similar to the to-be-crimped stent 1, e.g., PTFE, Nylon or PEBAX. The supporting mandrel 2 is of a solid, cylindrical structure, and the friction coefficient of its outer surface may be less than a predetermined threshold, so as to provide a smooth outer surface and enable the to-be-crimped stent 1 to be removed from the supporting mandrel 2 conveniently after the crimping. The determination of the friction coefficient is known in the art, and thus will not be particularly defined herein.

Preferably, as shown in Fig.4, similar to the selection of the supporting mandrel 2, the stopping pipe 3 may be made of a polymer material identical or similar to the to-be-crimped stent 1, e.g., PTFE, Nylon or PEBAX.

As shown in Fig.4, the to-be-crimped stent 1, the supporting mandrel 2 and the stopping pipe 3 are made of substantially the same material, and after the crimping, they are attached to each other tightly. In order to distinguish them from each other and remove the to-be-crimped stent 1 from the supporting mandrel 2 and the stopping pipe 3, preferably, axial lengths of the supporting mandrel 2 and the stopping pipe 3 may be greater than an axial length of the to-be-crimped stent 1.

It should be appreciated that, this crimping structure may refer to the above embodiments about the crimping method, so as to achieve the same technical effect.

The above are merely the preferred embodiments of the present disclosure. It should be appreciated that, a person skilled in the art may make further modifications and improvements without departing from the principle of the present disclosure, and these modifications and improvements shall also fall within the scope of the present disclosure.

## Claims

1. A crimping method for an endovascular stent, comprising steps of:
preparing a crimping structure which comprises a to-be-crimped stent (1), a supporting mandrel (2) arranged inside the to-be-crimped stent (1) and a stopping pipe (3) sleeved onto the to-be-crimped stent (1); and
crimping the crimping structure in accordance with a first crimping configuration, until the to-be-crimped stent (1) is attached to the supporting mandrel (2) and a force value from a crimper reaches a first value;
**characterised in that** the supporting mandrel (2) is of a solid, cylindrical structure.

2. The crimping method according to claim 1, wherein the step of preparing the crimping structure comprises:
selecting the supporting mandrel (2) and the stopping pipe (3) fit to the to-be-crimped stent (1) with a diameter of the supporting mandrel (2) being a percentage of a current diameter of the to-be-crimped stent (1); and
sleeving the to-be-crimped stent (1) onto the supporting mandrel (2), and sleeving the stopping pipe (3) onto the to-be-crimped stent (1) with the stopping pipe (3) being attached to the to-be-crimped stent (1).

3. The crimping method according to claim 1 or 2, wherein subsequent to crimping the crimping structure, the crimping method comprises:
removing the to-be-crimped stent which has been crimped between the supporting mandrel (2) and the stopping pipe (3), to obtain the to-be-crimped stent which has been crimped.

4. The crimping method according to claim 3, wherein subsequent to removing the to-be-crimped stent which has been crimped between the supporting mandrel (2) and the stopping pipe (3), the crimping method comprises:
sleeving the to-be-crimped stent which has been crimped onto a balloon catheter and sleeving another stopping pipe onto the to-be-crimped stent which has been crimped, so as to prepare a delivery structure; and
inflating the balloon catheter in accordance with a second crimping configuration, and crimping the delivery structure, until the to-be-crimped stent which has been crimped is secured onto the balloon catheter and the force value from the crimper reaches a second value.

5. The crimping method according to claim 4, wherein before sleeving the to-be-crimped stent which has been crimped onto the balloon catheter to prepare the delivery structure, the crimping method comprises:
repeating the steps of preparing a crimping structure and crimping the crimping structure, until the diameter of the to-be-crimped stent is reduced to a predetermined value.

6. The crimping method according to claim 3, wherein subsequent to crimping the delivery structure, the crimping method comprises:
removing the stopping pipe (3) from the to-be-crimped stent which has been crimped.

7. The crimping method according to claim 2, wherein the diameter of the supporting mandrel (2) is 60% to 90% of the diameter of the to-be-crimped diameter (1).

8. The crimping method according to claim 1, wherein the step of crimping the crimping structure in accordance with the first crimping configuration comprises:
setting the first crimping configuration for the crimper comprising a first preheating temperature, a first preheating duration, a first diameter of a crimping hole, a first diameter reduction rate of the crimping hole, the first value and a first form maintenance duration; and
crimping the crimping structure in accordance with the first crimping configuration.

9. The crimping method according to claim 8, wherein the first preheating temperature is 45 °C to 55 °C; the first preheating duration is 30s to 120s; the first diameter is identical to a current diameter of the to-be-crimped stent (1); the first diameter reduction rate is 0.01mm/s to 0.2mm/s; the first value is 10N to 60N; the first form maintenance duration is 30s to 120s.

10. The crimping method according to claim 4, wherein the steps of inflating the balloon catheter in accordance with the second crimping configuration and crimping the delivery structure comprise:
setting the second crimping configuration for the crimper comprising a second preheating temperature, a second preheating duration, a second diameter of a crimping hole, a second diameter reduction rate of the crimping hole, a filling pressure of the balloon catheter, the second value, and a second form maintenance duration; and
inflating the balloon catheter in accordance with the second crimping configuration and crimping the delivery structure.

11. The crimping method according to claim 10, wherein the second preheating temperature is 45 °C to 55 °C; the second preheating duration is 30s to 120s; the second diameter is identical to the current diameter of the to-be-crimped stent (1); the second diameter reduction rate is 0.01mm/s to 0.2mm/s, the filling pressure of the balloon catheter is 207kPa (30psi) to 1380kPa (200psi); the second value is 100N to 150N; the second form maintenance duration is 60s to 240s.

12. A crimping structure for an endovascular stent, comprising:
a tubular to-be-crimped stent (1);
a supporting mandrel (2) onto which the to-be-crimped stent (1) is sleeved; and
a stopping pipe (3) capable of being deformed by an external force, and sleeved onto and being attached to the to-be-crimped stent (1),
wherein a diameter of the supporting mandrel (2) is a percentage of a diameter of the to-be-crimped stent (1);
**characterized in that** the supporting mandrel (2) is of a solid, cylindrical structure.

13. The crimping structure according to claim 12, wherein the percentage is 60% to 90%.

14. The crimping structure according to claim 12, wherein the supporting mandrel (2) is made of polytetrafluoroethylene (PTFE), polyamide fiber (Nylon) or polyether block amide (PEBAX).

15. The crimping structure according to claim 12, wherein a friction coefficient of an outer surface of the supporting mandrel (2) is less than a threshold.

16. The crimping structure according to claim 12, wherein the stopping pipe (3) is made of PTFE, Nylon or PEBAX.

17. The crimping structure according to claim 12, wherein axial lengths of each of the supporting mandrel (2) and the stopping pipe (3) are greater than an axial length of the to-be-crimped stent (1).

## Patentansprüche

1. Crimpverfahren für einen endovaskulären Stent, wobei das Verfahren die folgenden Schritte umfasst:
Vorbereiten einer Crimpstruktur, die einen zu crimpenden Stent (1), einen in dem zu crimpenden Stent (1) angeordneten stützenden Dorn (29) und ein Abschaltrohrleitung (3), die auf den zu crimpenden Stent (1) geschoben ist; und
Crimpen der Crimpstruktur gemäß einer ersten Crimpkonfiguration, bis der zu crimpende Stent (1) an dem stützenden Dorn (29) angebracht ist und ein Kraftwert von einer Crimpeinrichtung einen ersten Wert erreicht;
**dadurch gekennzeichnet, dass** der stützende Dorn (2) eine feste, zylindrische Struktur aufweist.

2. Crimpverfahren nach Anspruch 1, wobei der Schritt des Vorbereitens der Crimpstruktur folgendes umfasst:
Auswählen des stützenden Dorns (2) und der Abschaltrohrleitung (3) passend zu dem zu crimpenden Stent (1), wobei ein Durchmesser des stützenden Dorns (2) ein prozentualer Anteil eines aktuellen Durchmessers des zu crimpenden Stents (1) ist; und
Schieben des zu crimpenden Stents (1) auf den stützenden Dorn (2), und Schieben der Abschaltrohrleitung (3) auf den zu crimpenden Stent (1), wobei die Abschaltrohrleitung (3) an dem zu crimpenden Stent (1) angebracht ist.

3. Crimpverfahren nach Anspruch 1 oder 2, wobei das Crimpverfahren nach dem Crimpen der Crimpstruktur folgendes umfasst:
Entfernen des zu crimpenden Stents, der zwischen dem stützenden Dorn (2) und der Abschaltrohrleitung (3) gecrimpt worden ist, um den gecrimpten zu crimpenden Stent zu erhalten.

4. Crimpverfahren nach Anspruch 3, wobei das Crimpverfahren nach dem Entfernen des zu crimpenden Stents, der zwischen dem stützenden Dorn (2) und der Abschaltrohrleitung (3) gecrimpt worden ist, folgendes umfasst:
Schieben des gecrimpten zu crimpenden Stents auf einen Ballonkatheter und Schieben einer weiteren Abschaltrohrleitung auf den gecrimpten zu crimpenden Stent, um eine Zufuhrstruktur vorzubereiten; und
Füllen des Ballonkatheters gemäß einer zweiten Crimpkonfiguration und Crimpen der Zufuhrstruktur, bis der gecrimpte zu crimpende Stent an dem Ballonkatheter gesichert ist und der Kraftwert von der Crimpeinrichtung einen zweiten Wert erreicht.

5. Crimpverfahren nach Anspruch 4, wobei das Crimpverfahren bevor der gecrimpte zu crimpende Stent auf den Ballonkatheter geschoben wird, um die Zufuhrstruktur vorzubereiten, folgendes umfasst:
Wiederholen der Schritte des Zubereitens einer Crimpstruktur und des Crimpens der Crimpstruktur, bis der Durchmesser des zu crimpenden Stents auf einen vorbestimmten Wert verringert ist.

6. Crimpverfahren nach Anspruch 3, wobei das Crimpverfahren nach dem Crimpen der Zufuhrstruktur folgendes umfasst:
Entfernen der Abschaltrohrleitung (3) von dem gecrimpten zu crimpenden Stent.

7. Crimpverfahren nach Anspruch 2, wobei der Durchmesser des stützenden Dorns (2) 60% bis 90% des Durchmessers des zu crimpenden Durchmessers (1) entspricht.

8. Crimpverfahren nach Anspruch 1, wobei der Schritt des Crimpens der Crimpstruktur gemäß der ersten Crimpkonfiguration folgendes umfasst:
Festlegen der ersten Crimpkonfiguration für die Crimpeinrichtung, wobei diese eine erste Vorerhitzungstemperatur, eine erste Vorerhitzungsdauer, einen ersten Durchmesser eines Crimplochs, eine erste Durchmesserverringerungsrate für das Crimploch, den ersten Wert und eine erste Form der Erhaltungsdauer umfasst; und
Crimpen der Crimpstruktur gemäß der ersten Crimpkonfiguration.

9. Crimpverfahren nach Anspruch 8, wobei die erste Vorerhitzungstemperatur 45 °C bis 55 °C beträgt; wobei die erste Vorerhitzungsdauer 30 Sekunden bis 120 Sekunden beträgt; wobei der erste Durchmesser mit einem aktuellen Durchmesser des zu crimpenden Stents (1) identisch ist; wobei die erste Durchmesserverringerungsrate 0,01 mm/Sekunde bis 0,2 mm/Sekunde beträgt; wobei der erste Wert zwischen 10 N und 60 N liegt; wobei die erste Form der Erhaltungsdauer 30 Sekunden bis 120 Sekunden beträgt.

10. Crimpverfahren nach Anspruch 4, wobei die Schritte des Füllens des Ballonkatheters gemäß der zweiten Crimpkonfiguration und das Crimpen der Zufuhrstruktur folgendes umfassen:
Festlegen einer zweiten Crimpkonfiguration für die Crimpeinrichtung, wobei diese eine zweite Vorerhitzungstemperatur, eine zweite Vorerhitzungsdauer, einen zweiten Durchmesser eines Crimplochs, eine zweite Durchmesserverringerungsrate, einen Fülldruck des Ballonkatheters, den zweiten Wert und eine zweite Form der Erhaltungsdauer umfasst; und
Füllen des Ballonkatheters gemäß der zweiten Crimpkonfiguration und Crimpen der Zufuhrstruktur.

11. Crimpverfahren nach Anspruch 10, wobei die zweite Vorerhitzungstemperatur 45 °C bis 55 °C beträgt; wobei der zweite Durchmesser mit dem aktuellen Durchmesser des zu crimpenden Stents (1) identisch ist; wobei die zweite Durchmesserverringerungsrate 0,01 mm/Sekunde bis 0,2 mm/Sekunde beträgt; wobei der Fülldruck des Ballonkatheters 207 kPa (30 psi) bis 1380 kPa (200 psi) beträgt; wobei der zweite Wert zwischen 100 N und 150 N liegt; wobei die zweite Form der Erhaltungsdauer 60 Sekunden bis 240 Sekunden beträgt.

12. Crimpstruktur für einen endovaskulären Stent, wobei die Struktur folgendes umfasst:
einen röhrenförmigen zu crimpenden Stent (1);
einen stützenden Dorn (2), auf welchen der zu crimpende Stent (1) geschoben wird; und
eine Abschaltrohrleitung (3), die durch eine externe Kraft verformt werden kann und die auf den zu crimpenden Stent (1) geschoben und an diesem angebracht werden kann;
wobei ein Durchmesser des stützenden Dorns (2) einem prozentualen Anteil eines Durchmessers des zu crimpenden Stents (1) entspricht;
**dadurch gekennzeichnet, dass** der stützende Dorn (2) eine feste, zylindrische Struktur aufweist.

13. Crimpstruktur nach Anspruch 12, wobei der prozentuale Anteil zwischen 60% und 90% liegt.

14. Crimpstruktur nach Anspruch 12, wobei der stützende Dorn (2) aus Polytetrafluorethylen (PTFE), Polyamidfaser (Nylon) oder Polyetherblockamid (PEBAX) besteht.

15. Crimpstruktur nach Anspruch 12, wobei ein Reibungskoeffizient einer äußeren Oberfläche des stützenden Dorns (2) niedriger ist als ein Schwellenwert.

16. Crimpstruktur nach Anspruch 12, wobei die Abschaltrohrleitung (3) aus PTFE, Nylon oder PEBAX besteht.

17. Crimpstruktur nach Anspruch 12, wobei die axialen Längen des stützenden Dorns (2) und der Abschaltrohrleitung (3) jeweils größer sind als eine axiale Länge des zu crimpenden Stents (1).

## Revendications

1. Procédé de sertissage d'un stent endovasculaire, comprenant les étapes consistant à :
préparer une structure de sertissage qui comprend un stent à sertir (1), un mandrin de support (2) disposé à l'intérieur du stent à sertir (1) et un tuyau d'arrêt (3) emmanché sur le stent à sertir (1) ; et
sertir la structure de sertissage selon une première configuration de sertissage, jusqu'à ce que le stent à sertir (1) soit fixé au mandrin de support (2) et qu'une valeur de force d'une sertisseuse atteigne une première valeur ;
**caractérisé en ce que** le mandrin de support (2) est d'une structure cylindrique solide.

2. Procédé de sertissage selon la revendication 1, la préparation de la structure de sertissage comprenant les étapes consistant à :
sélectionner le mandrin de support (2) et le tube d'arrêt (3) adaptés au stent à sertir (1), un diamètre du mandrin de support (2) étant un pourcentage d'un diamètre actuel du stent à sertir (1) ; et
emmancher le stent à sertir (1) sur le mandrin de support (2), et emmancher le tube d'arrêt (3) sur le stent à sertir (1), le tube d'arrêt (3) étant fixé au stent à sertir (1).

3. Procédé de sertissage selon la revendication 1 ou 2, après le sertissage de la structure de sertissage, le procédé de sertissage comprenant l'étape consistant à :
retirer le stent à sertir qui a été serti entre le mandrin de support (2) et le tube d'arrêt (3), pour obtenir le stent à sertir qui a été serti.

4. Procédé de sertissage selon la revendication 3, après le retrait du stent à sertir qui a été serti entre le mandrin de support (2) et le tube d'arrêt (3), le procédé de sertissage comprenant les étapes consistant à :
emmancher le stent à sertir qui a été serti sur un cathéter à ballonnet et emmancher un autre tube d'arrêt sur le stent à sertir qui a été serti, de sorte à préparer une structure de pose ; et
gonfler le cathéter à ballonnet selon une seconde configuration de sertissage, et sertir la structure de pose, jusqu'à ce que le stent à sertir qui a été serti soit fixé sur le cathéter à ballonnet et que la valeur de force de la sertisseuse atteigne une seconde valeur.

5. Procédé de sertissage selon la revendication 4, avant l'emmanchement du stent à sertir qui a été serti sur le cathéter à ballonnet pour préparer la structure de pose, le procédé de sertissage comprenant l'étape consistant à :
répéter la préparation d'une structure de sertissage et le sertissage de la structure de sertissage, jusqu'à ce que le diamètre du stent à sertir soit réduit à une valeur prédéfinie.

6. Procédé de sertissage selon la revendication 3, après le sertissage de la structure de pose, le procédé de sertissage comprenant l'étape consistant à :
retirer le tuyau d'arrêt (3) du stent à sertir qui a été serti.

7. Procédé de sertissage selon la revendication 2, le diamètre du mandrin de support (2) étant de 60 % à 90 % du diamètre du stent à sertir (1).

8. Procédé de sertissage selon la revendication 1, le sertissage de la structure de sertissage selon la première configuration de sertissage comprenant les étapes consistant à :
régler la première configuration de sertissage pour la sertisseuse comprenant une première température de préchauffage, une première durée de préchauffage, un premier diamètre d'un trou de sertissage, un premier taux de réduction du diamètre du trou de sertissage, la première valeur et une première durée de maintien de forme ; et
sertir la structure de sertissage selon la première configuration de sertissage.

9. Procédé de sertissage selon la revendication 8, la première température de préchauffage étant de 45 °C à 55°C ; la première durée de préchauffage étant de 30 s à 120 s ; le premier diamètre étant identique à un diamètre actuel du stent à sertir (1) ; le premier taux de réduction du diamètre étant de 0,01 mm/s à 0,2 mm/s ; la première valeur étant de 10 N à 60 N ; la première durée de maintien de forme étant de 30 s à 120 s.

10. Procédé de sertissage selon la revendication 4, le gonflage du cathéter à ballonnet selon la seconde configuration de sertissage et le sertissage de la structure de pose comprenant les étapes consistant à :
régler la seconde configuration de sertissage pour la sertisseuse comprenant une seconde température de préchauffage, une seconde durée de préchauffage, un second diamètre d'un trou de sertissage, un second taux de réduction du diamètre du trou de sertissage, une pression de remplissage du cathéter à ballonnet, la seconde valeur et une seconde durée de maintien de forme ; et
gonfler le cathéter à ballonnet conformément à la seconde configuration de sertissage et sertir la structure de pose.

11. Procédé de sertissage selon la revendication 10, la seconde température de préchauffage étant de 45 °C à 55°C ; la seconde durée de préchauffage étant de 30 s à 120 s ; le second diamètre étant identique à un diamètre actuel du stent à sertir (1) ; le second taux de réduction du diamètre étant de 0,01 mm/s à 0,2 mm/s ; la pression de remplissage du cathéter à ballonnet étant de 207 kPa (30 psi) à 1 380 kPa (200 psi) ; la seconde valeur étant de 100 N à 150 N ; la seconde durée de maintien de forme étant de 60 s à 240 s.

12. Structure de sertissage pour un stent endovasculaire, comprenant :
un stent tubulaire à sertir (1) ;
un mandrin de support (2) sur lequel le stent à sertir (1) est emmanché ; et
un tuyau d'arrêt (3) pouvant être déformé par une force extérieure, et emmanché sur et fixé au stent à sertir (1),
un diamètre du mandrin de support (2) étant un pourcentage d'un diamètre du stent à sertir (1) ;
**caractérisé en ce que** le mandrin de support (2) est d'une structure cylindrique solide.

13. Structure de sertissage selon la revendication 12, le pourcentage étant de 60 % à 90%.

14. Structure de sertissage selon la revendication 12, le mandrin de support (2) étant constitué de polytétrafluoroéthylène (PTFE), de fibres de polyamide (Nylon) ou de polyéther bloc amide (PEBAX).

15. Structure de sertissage selon la revendication 12, un coefficient de frottement d'une surface externe du mandrin de support (2) étant inférieure à un seuil.

16. Structure de sertissage selon la revendication 12, le tube d'arrêt (3) étant en PTFE, Nylon ou PEBAX.

17. Structure de sertissage selon la revendication 12, les longueurs axiales de chacun des mandrins de support (2) et du tube d'arrêt (3) étant supérieures à une longueur axiale du stent à sertir (1).
